(19) **Europäisches Patentamt / European Patent Office / Office européen des brevets**

(11) **EP 2 455 358 A1**

(12) **EUROPEAN PATENT APPLICATION**
published in accordance with Art. 153(4) EPC

(43) Date of publication:
**23.05.2012 Bulletin 2012/21**

(21) Application number: **10799355.2**

(22) Date of filing: **15.07.2010**

(51) Int Cl.:
*C07C 2/60* (2006.01)  *C10G 50/00* (2006.01)
*B01J 38/54* (2006.01)  *B01J 38/48* (2006.01)
*B01J 31/02* (2006.01)

(86) International application number:
**PCT/CN2010/001066**

(87) International publication number:
**WO 2011/006357 (20.01.2011 Gazette 2011/03)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **17.07.2009 CN 200910089442**

(71) Applicant: **Shell Internationale Research Maatschappij B.V.**
**2596 HR The Hague (NL)**

(72) Inventors:
• **LIU, Zhichang**
  **Changping**
  **Beijing 102249 (CN)**

• **XU, Chunming**
  **Changping**
  **Beijing 102249 (CN)**
• **ZHANG, Rui**
  **Changping**
  **Beijing 102249 (CN)**
• **MENG, Xianghai**
  **Changping**
  **Beijing 102249 (CN)**

(74) Representative: **Matthezing, Robert Maarten et al**
  **Shell International B.V.**
  **Intellectual Property Services**
  **P.O. Box 384**
  **2501 CJ The Hague (NL)**

(54) **METHODS FOR REGENERATING AND MAINTAINING ACTIVITY OF IONIC LIQUID CATALYST AND PRODUCING ALKYLATE**

(57)    Disclosed is a method for regenerating and maintaining the activity of an ionic liquid catalyst, which comprises supplying hydrogen halide or halogenated hydrocarbon to the acidic ionic liquid catalyst or alkylation materials during alkylation reaction, wherein said ionic liquid catalyst is used to catalyze alkylation of C4 alkene and alkane. Disclosed is also a method for producing alkylate by alkylation reaction, which comprises supplying hydrogen halide or halogenated hydrocarbon to the acidic ionic liquid catalyst or reaction materials during said alkylation reaction. The method can prolong the service life of the acidic ionic liquid catalyst, does not influence the quality of the alkylate, and has simple operation. The processed amount of materials may be 1000 times more than the used amount of the ionic liquid.

Fig. 1

**Description**

Field of the invention

The present invention is related to a process for regenerating catalyst, more specifically to a process for regenerating and maintaining the activity of an ionic liquid catalyst so that producing continuously alkylate. The present invention belongs to the petrochemical field.

Background of the invention

In petroleum refining industry, the alkylation reaction between alkane and alkene (also known as C4 alkylation, normally using more isobutane and also know as isobutane alkylation) is an important process for producing clean high-octane gasoline blending component. The alkylate obtained from said alkylation process is a fuel product, i.e. a liquid product derived from the catalytic reaction between C4 alkene and alkane by an acidic catalyst. In other words, it is a particular alkylation product, wherein C8 selectivity and TMP/DMH (trimethylpentane/dimethylhexane) ratio are important to evaluate the quality of alkylate product.

Hydrofluoric acid and concentrated sulfuric acid are conventional industrial catalysts for producing alkylate by alkylation reaction. Both of them exhibit satisfactory properties in terms of the activity, the selectivity and the catalyst lifetime, however, they may result in some problems such as environmental pollution, equipments corrosion as well as the personnels injuries etc. simultaneously, thus, the industrial development of C4 alkylation being limited seriously.

As a novel compound system, ionic liquids have the properties such as being environmental friendly, less corrosive, low toxicity, adjustable acidity and physical-chemical properties, being easily separated from the product as well as being recycled at high rate, so that to become a novel desired catalytic material to be used to catalyze C4 instead of those liquid strong acids such as hydrofluoric acid and concentrated sulfuric acid. Regarding utilizing acidic ionic liquids as catalysts for the alkylation reaction between isoalkane and alkene, there are already lots of reports, which primarily focused on how to choose and prepare the relevant ionic liquid catalysts in order to improve the catalytic activity and accelerate the alkylation reaction. US 7285698, US 20040133056A1 and CN 02149296.4 further disclosed the processes for catalyzing the alkylation reaction between isobutane and butene using a composite ionic liquid, of which the anion is derived from two or more metallic compounds, as catalyst, wherein the yield of the alkylation product-- alkylate may be up to 170-180% by volume of the alkene feed by selecting the structure of the composite ionic liquid, C8 fractions may be 60-80% of the alkylate, most prominently trimethylpentane may be more than 70% of C8 fractions, and RON(research octane number) may be of 93-98.

No matter simple ionic liquid, of which the anion is derived from a single metallic compound, or composite ionic liquid is used as the catalyst in the catalytic alkylation reaction, the issue about the catalyst being deactivated can not be avoided. The feed processed by the ionic liquid catalyst can hardly be more than 100 g per gram of the ionic liquid, thus, the ionic liquid catalyst should be replaced and regenerated frequently and the corresponding industrial process would be limited apparently. There are also some researches providing technical solutions regarding the reasons they thought why the ionic liquid catalyst being deactivated. For instance, Chinese patent application 200710063459.6 disclosed a process for prolong the catalyst life by using metallic aluminum or aluminum trichloride as aluminum source to compensate the aluminum trichloride lost due to entrainment in oil phase and hydrolysis of ionic liquid in the presence of water. The inventors of this application found that the primary reason for chloroaluminate as ionic liquid catalyst being deactivated is the loss of the active aluminum trichloride therein decreases the reaction activity, and the loss of aluminum trichloride is primarily due to the hydrolysis of aluminum trichloride caused by the inevitable water in the reaction feed and further possibly due to that aluminum trichloride may be entrained into oil phase by some strong electron-donating species, which may be present in the reaction system. Thus, said patent application provided a process for producing alkylate, wherein the ionic liquid catalyst is regenerated on line continuously by using metallic aluminum or aluminum trichloride as aluminum source to compensate the aluminum trichloride lost due to entrainment in oil phase and hydrolysis of ionic liquid so as to makeup aluminum continuously and extend the catalyst life. Said patent application further stated that the process can also reduce the amount of HCl present in the catalytic reaction system, thus maintaining the selectivity of the targeted isooctane in the product at high level and reducing the corrosion due to the presence of HCl. However, a series of patent documents such as Chinese patent application CN 200680051282.1, CN 200680052353.X, United States Patent 20070142211 and 20070142214 disclosed processes for regenerating ionic liquid catalysts used in alkylation reaction by removing mixed polymers because it is believed that the ionic liquid catalysts are deactivated due to their anionic components being deactivated by the mixed polymers.

Therefore, it is important to effectively obviate the deactivation of the ionic liquids for spreading and promoting the commercial uses of ionic liquids as catalysts in the production of alkylate.

Summary of the invention

The major technical problem to be solved by the present invention is to provide a process for effectively regenerating and maintaining the catalytic activity of ionic liquid catalyst based on the mechanism of catalyzing alkylation reaction by an acidic ionic liquid, so as to effectively prolong the useful life of the acidic ionic liquid catalysts.

The present invention further provides a process for producing alkylate by alkylation reaction using acidic ionic liquid as catalyst, wherein during the alkylation reaction by supplying hydrogen halide or halogenated hydrocarbon, the useful life of the acidic ionic liquid catalyst may be prolonged effectively and the amount of the feed to be processed by per mass of the ionic liquid may be increased, and said process can easily be operated without negative effects on the quality of the alkylate, thus may be used commercially.

The present invention provides a process for regenerating and maintaining the catalytic activity of ionic liquid catalyst used as catalyst in alkylation reaction for producing alkylate, characterized by supplying hydrogen halide or halogenated hydrocarbon to acidic ionic liquid catalyst or reaction feed during alkylation reaction.

The catalytic alkylation reaction by acidic ionic liquid catalyst is following the positively charged carbon ion mechanism, wherein both the Lewis acid and Brönsted acid of the ionic liquid function together during the catalytic reaction, the acidity of Lewis acid of the ionic liquid primarily determines the product selectivity, and the acidity of Brönsted acid of the ionic liquid primarily determines whether or not the positively charged carbon ion may be generated. The present inventors found that the alkylate product, comprising no isobutane at excessive amount, normally comprises 0.01 ~ 1wt% of halogen during the catalytic alkylation by ionic liquid, resulting in gradual loss of Brönsted acid with the reaction proceeding, thus, the ionic liquid being deactivated. Therefore, the process according to the present invention comprises regenerating the acidity of Brönsted acid of the ionic liquid. It has been demonstrated to be an effective approach to extend the lifetime of the ionic liquid.

Based on the above facts, the process according to the present invention requires controlling the supplied amount of hydrogen halide or halogenated hydrocarbon within a reasonable range based on the produced alkylate, preferably being 0.01-1wt% of the produced alkylate calculated as the mass of the halogen contained therein.

The above mentioned process according to the present invention is suitable for regenerating the acidic ionic liquid catalysts used in a variety of alkylation reactions, especially the acidic ionic liquid catalyst used in alkylation for producing alkylate. Therefore, the present invention further provides a process for producing alkylate by alkylation reaction using isobutane and C4 alkene as reaction feed and acidic ionic liquid as catalyst, said process is characterized by supplying hydrogen halide or halogenated hydrocarbon to the ionic liquid catalyst or the reaction feed during the alkylation reaction to regenerate said acidic ionic liquid catalyst.

Producing alkylate by alkylation reaction has already become a highly concerned and intensively investigated technology in the art. There are lots of related patents published prior to present invention regarding the studies and improvements of this technology, especially the improvement of the ionic liquid catalysts used therein. For example, the above cited documents about the processes for producing alkylate utilizing composite ionic liquid as catalyst further described the effect of said composite ionic liquid catalyst on the alkylation between C4 alkene and isobutane based on those disclosed prior art. In fact, the present invention is proposed based on these disclosed prior art related to alkylation reaction as well as preparation of alkylate, and is primarily related to regenerating and maintaining the catalytic activity of acidic ionic liquid catalysts used in the art by suitably supplying hydrogen halide or halogenated hydrocarbon during the well known alkylation process. No further descriptions are mentioned herein regarding the preparation of ionic liquid catalyst as well as the alkylation reaction conditions and apparatuses. The disclosures of United States Patent 7285698, 20040133056A1 and Chinese patent 02149296.4 are incorporated herein by reference in their entirety.

Preferably, during the alkylation reaction according to the present invention, the acidic ionic liquid has a cation derived from hydrohalide of alkyl amine, hydrohalide of imidazole or hydrohalide of pyridine and an anion derived from one or more metallic compounds.

According to the process of the present invention, said acidic ionic liquid may have anion derived from two or more metallic compounds, of which at least one metallic compound is aluminum chloride or aluminum bromide and the other metallic compounds are halide, sulphate or nitrate of copper, iron, zinc, nickel, titanium or silver. Within the anion portion the molar ratio between aluminum compound and other metallic compounds is in the range of 1:100-100:1, preferably in the range of 1:1-100:1, more preferably in the range of 5:1-50:1. According to the process of the present invention, the reaction feed for the alkylation reaction essentially is a mixture of isobutane and C4 alkene, wherein C4 alkene comprises various iso- and n-alkenes such as 2-butene, iso-butene, 1-butene or mixture thereof, and in practical production the feed may comprise propylene at a minor amount; the isobutane is normally required at an excessive amount during the preparation and may also comprise propane, pentane and alkane with other carbon number at a minor amount, i.e. the molar ratio between alkane and alkene in the feed should be more than 1, normally 1:1-40:1; the reaction temperature may be in the range of 20-100°C, preferably in the range of 0-50°C; the reaction pressure should maintain the reaction feed as liquids under the reaction conditions, normally in the range of 0.1-1.6MPa; and the ionic liquid is regenerated in such manners that the reaction efficiency may be increased, the reaction cost may be reduced, and at the same time the selectivity of the alkylation reaction as well as the yields of the products may be improved according to the present invention.

According to the present invention, in order to regenerate the acidic ionic liquid and maintain the activity thereof, the supplied hydrogen halide is hydrogen chloride or hydrogen bromide, and the halogenated hydrocarbon is chlorohydrocarbon or bromohydrocarbon comprising at least 4 carbons. Preferably, in the structure of the halogenated hydrocarbon, the carbon having halogen atom attached thereto may be secondary or tertiary carbon, preferably tertiary carbon. Preferably, the halogenated hydrocarbon is halogenated alkane having the above mentioned structure, especially the halogenated alkane having 4-8 carbons, for instance, 2-chloro (bromo)-2-methyl-propane, 2-chloro (bromo)-2-methyl-hexane, 2-bromo-butane and 2-chloro-butane etc. may be used.

The present invention provide a process, which may carry out alkylation reaction continuously, wherein isobutane and C4 alkene being used as feed and any suitable acidic ionic liquids as above mentioned being used as catalyst, during the catalytic alkylation reaction, the ionic liquid catalyst is regenerated and the alkylate is produced continuously by supplying hydrogen halide or halogenated hydrocarbon to said acidic ionic liquid catalytic system. According to practical production the hydrogen halide or halogenated hydrocarbon may be supplied in batch, semicontinuously or continuously.

During the alkylation of the present invention, supplying hydrogen halide or halogenated hydrocarbon to the acidic ionic liquid catalyst or the reaction feed may comprise the following steps:

(i) Stopping feeding after the catalytic alkylation reaction using the acidic ionic liquid is proceeding for a period;
(ii) Supplying hydrogen halide or halogenated hydrocarbon to the acidic ionic liquid catalyst and intensively mixing the same;
(iii) Continuing the catalytic alkylation reaction using the acidic ionic liquid with hydrogen halide or halogenated hydrocarbon supplied thereinto and repeating steps (i) and (ii).

Regarding to above mentioned batch process, considering about the regeneration of the acidic ionic liquid, the alkylation reaction may be stopped theoretically when the ionic liquid gets deactivated partly or fully, or the alkylation reaction may be stopped prior to the ionic liquid gets deactivated at least partly and then the catalyst may be regenerated. However, in practical production, in order to ensure the selectivity of C8 fractions of the alkylate and avoid introducing the alkenes, the regeneration may be carried out prior to the ionic liquid gets deactivated based on the production process. According to the present invention, whether the ionic liquid getting deactivated or not is determined based on the alkene conversion during the alkylation reaction, i.e. the catalyst is believed being deactivated fully if the alkene conversion is 0, the catalyst is believed being active if the alkene conversion is 100%, and the catalyst is believed being deactivated partly if the conversion is between 0 and 100%.

During the alkylation of the present invention, supplying hydrogen halide or halogenated hydrocarbon to the acidic ionic liquid catalyst or the reaction feed may comprise the following step:

Continuously supplying hydrogen halide or halogenated hydrocarbon to the acidic ionic liquid catalyst or the reaction feed while the alkylation reaction is proceeding.

During the alkylation of the present invention, supplying hydrogen halide or halogenated hydrocarbon to the acidic ionic liquid catalyst or the reaction feed may comprise the following steps:

(i) Separating a part of the acidic ionic liquid catalyst while the catalytic alkylation reaction using the acidic ionic liquid catalyst is proceeding;
(ii) Supplying hydrogen halide or halogenated hydrocarbon to the separated acidic ionic liquid catalyst and intensively mixing the same;
(iii) Reinjecting the acidic ionic liquid with hydrogen halide or halogenated hydrocarbon supplied thereinto back to the reaction system and repeating steps (i) and (ii).

Wherein, all of steps (i), (ii) and (iii) may be batch or continuous.

Similarly, said process may be referred as semicontinuous process. With regard to the catalyst separated from the reaction system, a "part" of catalyst separated from the reaction system may be theoretically of 1-99% of the total catalysts in the reaction system with respect to various feed rate, and in the practical process design, the catalyst separated from the reaction system and to be regenerated preferably is of 1-50% of the total catalyst in the reaction system.

Brief description of the Drawings

Figure 1 is a schematic representative depicting an embodiment for regenerating and maintaining the activity of the ionic liquid catalyst by supplying in batch or continuously hydrogen halide or halogenated hydrocarbon according to the present invention.

Figure 2 is a schematic representative depicting an embodiment for regenerating and maintaining the activity of the ionic liquid catalyst by supplying semicontinuously hydrogen halide or halogenated hydrocarbon according to the present invention.

Detailed Description

Now the present invention and the advantageous effects thereof will be further illustrated by following examples, which should not be construed as limitations to the scope of the present invention.

The acidic ionic liquid catalysts used in the examples are synthesized according to the processes described in United States Patent 7285698, 20040133056A1 and Chinese patent 02149296.4 or purchased commercially. The composition of the alkylate product is determined by gas chromatograph and the activity of the catalysts is based on the butene conversions in the examples. The butene conversion is defined as following:

$$\text{Conversion} = ((\text{the initial butene mass} - \text{the butene mass after reaction})/ \text{the initial butene mass}) \times 100\%$$

1. Supplying hydrogen halide or halogenated hydrocarbon in batch to carry out alkylation reaction for producing alkylate

Example 1:

Catalytic alkylation reaction of isobutane is carried out in a continuous apparatus using acidic ionic liquid as catalyst, wherein the cation is provided by $Et_3NHC1$ and the anion is provided by $AlCl_3$ and CuCl. The ionic liquid is added at an amount of 200g. The reaction pressure is 0.5MPa and the reaction temperature is 25 °C. The feed is a mixture of isobutane and 2-butene with a molar ratio between alkane and alkene of 20:1. Collecting the alkylation product obtained from the catalytic alkylation reaction by fresh catalyst and analysing composition thereof, and the results are shown in table 1.

As seen from table 1, when the feed processed by per gram of the ionic liquid catalyst is up to 50 g, the catalyst activity declines significantly. When the feed processed by per gram of the ionic liquid catalyst is up to 60 g, the butene conversion is almost of 0, which indicates the catalyst gets deactivated fully. The obtained alkylate product is of 1120g in all.

Table 1 Continuous evaluation on catalytic alkylation by the fresh ionic liquid

| Processed feed, g/g ionic liquid | Butene conversion, % | Selectivity of C8 fractions, wt% | TMP/DMH |
|---|---|---|---|
| 10 | 100 | 85 | 12 |
| 20 | 100 | 86 | 14 |
| 30 | 100 | 86 | 14 |
| 40 | 100 | 86 | 14 |
| 50 | 76 | 80 | 13 |
| 60 | 0 | - | - |

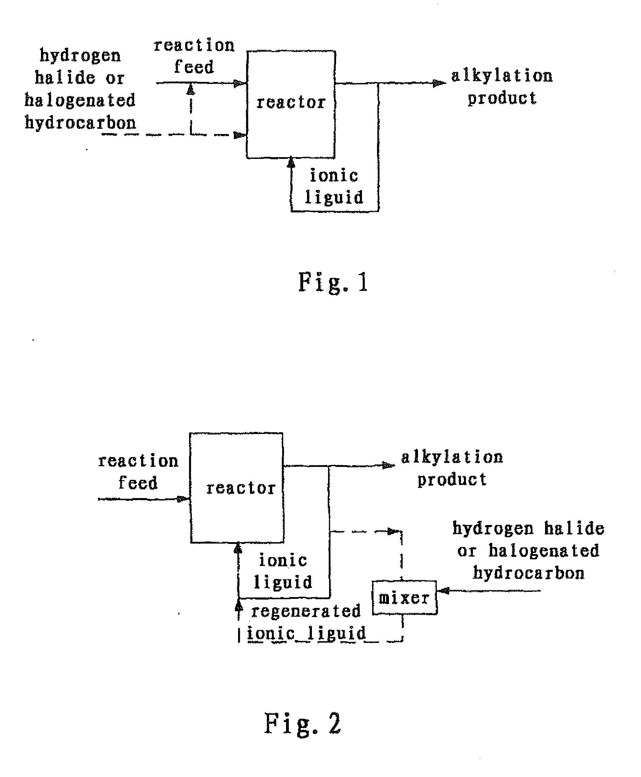

As shown in Fig.1, after confirming that the catalyst is fully deactivated, stopping feeding to the alkylation reaction. Separating the ionic liquid from the alkylation product and reinjecting the same back to the reactor, and supplying 15g of 2-chloro-2-methyl hexane to the reactor simultaneously and intensively mixing with the deactivated ionic liquid therein. When completed, keeping the above mentioned reaction conditions, continuing feeding reaction feed to the reaction system, collecting the alkylation product catalyzed by the regenerated catalyst and analysing composition thereof, and the results are shown in table 2. As can be seen, the activity of the ionic liquid was regenerated and the selectivity of the targeted product (C8 fractions) changed a little.

Table 2 Continuous evaluation on catalytic alkylation by the regenerated ionic liquid

| Processed feed, g/g ionic liquid | Butene conversion, % | Selectivity of C8 fractions, wt% | TMP/DMH |
|---|---|---|---|
| 10 | 100 | 85 | 14 |
| 20 | 100 | 85 | 14 |
| 30 | 100 | 87 | 15 |
| 40 | 100 | 87 | 15 |
| 50 | 100 | 87 | 15 |
| 60 | 54 | 77 | 13 |
| 70 | 0 | - | - |

After repeating above step 10 times using the ionic liquid, evaluating the alkylation reaction under the same reaction conditions, and the results are shown in table 3.

Both the activity of the regenerated ionic liquid catalyst and the selectivity of C8 fractions changed a little. It can be anticipated that the ionic liquid catalyst could be repeatedly processed and used in such manners.

Table 3 Continuous evaluation on catalytic alkylation by the ionic liquid being regenerated 10th time

| Processed feed, g/g ionic liquid | Butene conversion, % | Selectivity of C8 fractions, wt% | TMP/DMH |
|---|---|---|---|
| 10 | 100 | 86 | 14 |
| 20 | 100 | 87 | 15 |
| 30 | 100 | 87 | 15 |
| 40 | 100 | 87 | 15 |
| 50 | 100 | 87 | 15 |
| 60 | 39 | 71 | 12 |
| 70 | 0 | - | - |

Example 2:

Catalytic alkylation reaction of isobutane is carried out in a continuous apparatus using acidic ionic liquid as catalyst, wherein the cation is provided by [bmim]Cl (butyl methyl imidazole chloride) and the anion is provided by $AlBr_3$ and $CuSO_4$. The ionic liquid is added at an amount of 200g. The reaction pressure is 0.5MPa and the reaction temperature is 20°C. The feed is a mixture of isobutane, 2-butene, iso-butene and 1-butene with a molar ratio between alkane and alkene of 40:1. Collecting the alkylation product obtained from catalytic alkylation reaction by fresh catalyst and analysing the composition thereof, and the results are shown in table 4.

As can be seen, when the feed processed by per gram of the ionic liquid catalyst is up to 100, the catalyst activity declines significantly. When the feed processed by per gram of the ionic liquid catalyst is up to 110 grams, the catalyst gets deactivated fully, and the obtained alkylate product is of 1121 g in all.

Table 4 Continuous evaluation on catalytic alkylation by the fresh ionic liquid

| Processed feed, g/g ionic liquid | Butene conversion, % | Selectivity of C8 fractions, wt% | TMP/DMH |
|---|---|---|---|
| 20 | 100 | 81 | 13 |
| 40 | 100 | 83 | 13 |
| 60 | 100 | 84 | 14 |
| 80 | 100 | 85 | 14 |
| 100 | 42 | 80 | 13 |
| 110 | 0 | - | - |

Same as that in example 1, after confirming that the ionic liquid is fully deactivated, stopping feeding to the alkylation reaction. Supplying 2g hydrogen bromide to the reactor and intensively mixing with the deactivated ionic liquid therein. When the mixing being completed, keeping the above mentioned reaction conditions, continuing feeding to the reaction system, collecting the alkylation product catalyzed by the regenerated catalyst and analysing composition thereof, and the results are shown in table 5.

As can be seen, the activity of the ionic liquid was regenerated and the selectivity of the targeted product C8 fractions changed a little.

Table 5 Continuous evaluation on catalytic alkylation by the regenerated ionic liquid

| Processed feed, g/g ionic liquid | Butene conversion, % | Selectivity of C8 fractions, wt% | TMP/DMH |
|---|---|---|---|
| 20 | 100 | 83 | 14 |
| 40 | 100 | 84 | 14 |
| 60 | 100 | 85 | 15 |
| 80 | 100 | 85 | 15 |
| 100 | 71 | 81 | 13 |
| 110 | 0 | - | - |

After repeating above step 10 times using the ionic liquid, evaluating the alkylation reaction under the same reaction conditions, and the results are shown in table 6. As can be seen, both the activity of the ionic liquid and the selectivity changed a little. It can be anticipated that the ionic liquid catalyst can be repeatedly processed and used in such manners.

Table 6 Continuous evaluation on catalytic alkylation by the ionic liquid being regenerated 10th time

| Processed feed, g/g ionic liquid | Butene conversion, % | Selectivity of C8 fractions, wt% | TMP/DMH |
|---|---|---|---|
| 20 | 100 | 84 | 14 |
| 40 | 100 | 84 | 15 |
| 60 | 100 | 85 | 15 |
| 80 | 100 | 85 | 15 |
| 100 | 65 | 81 | 14 |
| 110 | 0 | - | - |

Noted: During the production of alkylate, determining the feed amount based on the loaded catalyst and regenerating the catalyst prior to the catalyst getting deactivated, so as to ensure the selectivity of C8 fractions in the alkylate product.

II. Continuously supplying hydrogen halide or halogenated hydrocarbon to carry out the alkylation reaction for producing alkylate continuously

Example 3:

Referring to the flow diagram depicted in Fig.1, catalytic alkylation reaction of isobutane is carried out in a continuous apparatus using acidic ionic liquid as catalyst, wherein the cation is provided by [bmim]Cl and the anion is provided by $AlCl_3$, The ionic liquid is added at an amount of 30kg. The reaction pressure is 0.5MPa and the reaction temperature is 30°C. The reaction feed is a mixture of isobutane, 2-butene, iso-butene and 1-butene with a molar ratio between alkane and alkene of 10:1. The feed flowrate is of 12kg/h, the alkylate is produced at a rate of 2.4kg/h, and the hydrogen chloride is supplied to the reactor at a rate of 2g/h continuously at the same time (or supplied to the reactor after being mixed with the reaction feed). Collecting the alkylation product and analysing the composition thereof, and the results are shown in table 7.

As can be seen from table 7, when the feed processed by per kilogram of the ionic liquid catalyst is up to 1000 kg, the catalyst is still not deactivated and the selectivity of the targeted product C8 fractions keeps unchanged.

Table 7 Continuous evaluation on catalytic alkylation by the ionic liquid with hydrogen chloride supplied thereinto continuously

| Processed feed, kg/kg ionic liquid | Butene conversion, % | Selectivity of C8 fractions, wt% | TMP/DMH |
|---|---|---|---|
| 200 | 100 | 80 | 11 |
| 400 | 100 | 81 | 12 |
| 600 | 100 | 80 | 12 |
| 800 | 100 | 80 | 12 |

(continued)

| Processed feed, kg/kg ionic liquid | Butene conversion, % | Selectivity of C8 fractions, wt% | TMP/DMH |
|---|---|---|---|
| 1000 | 100 | 80 | 12 |

Comparative Example 1:

Also referring to the flow diagram depicted in Fig.1, catalytic alkylation reaction of isobutane is carried out in a continuous apparatus using acidic ionic liquid as catalyst, wherein the cation is provided by [bmim]Cl and the anion is provided by $AlCl_3$. The ionic liquid is added at an amount of 30kg. The reaction pressure is 0.5MPa and the reaction temperature is 30°C. The reaction feed is a mixture of isobutane, 2-butene, iso-butene and 1-butene with a molar ratio between alkane and alkene of 10:1. The feed flowrate is of 12kg/h, the alkylate is produced at a rate of 2.4kg/h, and the hydrogen chloride is supplied to the reactor at a rate of 30 g/h continuously at the same time (or supplied to the reactor after being mixed with the reaction feed). Collecting the alkylation product and analysing the composition thereof, and the results are shown in table 8. As can be seen, when the feed processed by per kilogram of the ionic liquid catalyst is up to 1000 kg, the catalyst is not deactivated but the selectivity of the targeted product C8 fractions is significantly declined compared with Example 3.

Table 8 Continuous evaluation on catalytic alkylation by the ionic liquid with hydrogen chloride supplied thereinto continuously

| Processed feed, kg/kg ionic liquid | Butene conversion, % | Selectivity of C8 fractions, wt% | TMP/DMH |
|---|---|---|---|
| 200 | 100 | 70 | 8 |
| 400 | 100 | 63 | 7 |
| 600 | 100 | 61 | 7 |
| 800 | 100 | 61 | 7 |
| 1000 | 100 | 60 | 7 |

Example 4:

Referring to the flow diagram depicted in Fig. 1, catalytic alkylation reaction of isobutane is carried out in a continuous apparatus using acidic ionic liquid as catalyst, wherein the cation is provided by $Et_3NHCl$ and the anion is provided by $AlCl_3$ and CuCl. The ionic liquid is added at an amount of 200g. The reaction pressure is 0.5MPa and the reaction temperature is 20°C. The reaction feed is a mixture of isobutane and 2-butene with a molar ratio between alkane and alkene of 20:1. The feed flowrate is of 500g/h, the alkylate is produced at a rate of 50g/h, and 2-bromo-butane is supplied to the reactor at a rate of 0.2g/h continuously at the same time (or supplied to the reactor after being mixed with the reaction feed). Collecting the alkylation product and analysing the composition thereof, and the results are shown in table 9. As can be seen, when the feed processed by per gram of the ionic liquid catalyst is up to 1000 g, the catalyst is still not deactivated and the selectivity of the targeted product C8 fractions keeps unchanged.

Table 9 Continuous evaluation on catalytic alkylation by the ionic liquid with 2-bromo-butane supplied thereinto continuously

| Processed feed, g/g ionic liquid | Butene conversion, % | Selectivity of C8 fractions, wt% | TMP/DMH |
|---|---|---|---|
| 200 | 100 | 85 | 14 |
| 400 | 100 | 86 | 15 |
| 600 | 100 | 87 | 15 |
| 800 | 100 | 87 | 15 |
| 1000 | 100 | 87 | 15 |

Example 5:

Referring to the flow diagram depicted in Fig.1, catalytic alkylation reaction of isobutane is carried out in a continuous apparatus using acidic ionic liquid as catalyst, wherein the cation is provided by [bmim]Cl and the anion is provided by $AlBr_3$ and $CuSO_4$. The ionic liquid is added at an amount of 200g. The reaction pressure is 0.5MPa and the reaction temperature is 20°C. The reaction feed is a mixture of isobutane, 2-butene and 2-chloro-2-methyl propane with a molar ratio between alkane and alkene of 20:1. The 2-chloro-2-methyl propane is added to the feed at a rate of 0.02wt% of the total feed weight. The feed flowrate is of 500g/h, and the alkylate is produced at a rate of 50g/h. Collecting the alkylation product and analysing the composition thereof, and the results are shown in table 10. As can be seen, when the feed processed by per gram of the ionic liquid catalyst is up to 1000 g, the catalyst is still not deactivated and the selectivity of the targeted product C8 fractions keeps unchanged.

Table 10 Continuous evaluation on catalytic alkylation by the ionic liquid with 2-chloro-2-methyl propane supplied thereinto continuously

| Processed feed, g/g ionic liquid | Butene conversion, % | Selectivity of C8 fractions, wt% | TMP/DMH |
|---|---|---|---|
| 200 | 100 | 86 | 14 |
| 400 | 100 | 86 | 15 |
| 600 | 100 | 87 | 15 |
| 800 | 100 | 87 | 15 |
| 1000 | 100 | 87 | 15 |

III. Semi-continuously supplying hydrogen halide or halogenated hydrocarbon to carry out the alkylation reaction for producing alkylate continuously

Example 6:

Referring to the flow diagram depicted in Fig.2, catalytic alkylation reaction of isobutane is carried out in a continuous apparatus using acidic ionic liquid as catalyst, wherein the cation is provided by $Et_3NHCl$ and the anion is provided by $AlBr_3$. The ionic liquid is added at an amount of 30kg. The reaction pressure is 0.5MPa and the reaction temperature is 30°C. The reaction feed is a mixture of isobutane, 2-butene, iso-butene and 1-butene with a molar ratio between alkane and alkene of 10:1. The feed flowrate is of 12kg/h, and the alkylate is produced at a rate of 2.4kg/h.
The ionic liquid is separated from the reaction system at an amount of 3kg every hour regularly and supplied into a mixer, wherein being intensively mixed with 2g hydrogen bromide and then being reinjected back to the reaction system. This procedure can be carried out in batch or continuously.
Collecting the alkylation product and analysing the composition thereof, and the results are shown in table 11. When the feed processed by per kilogram of the ionic liquid catalyst is up to 1000 kg, the catalyst is still not deactivated and the selectivity of the targeted product C8 fractions keeps unchanged.

Table 11 Continuous evaluation on catalytic alkylation by the ionic liquid with hydrogen bromide supplied thereinto semi-continuously

| Processed feed, kg/kg ionic liquid | Butene conversion, % | Selectivity of C8 fractions, wt% | TMP/DMH |
|---|---|---|---|
| 200 | 100 | 81 | 12 |
| 400 | 100 | 82 | 12 |
| 600 | 100 | 82 | 13 |
| 800 | 100 | 82 | 13 |
| 1000 | 100 | 82 | 13 |

Example 7:

Catalytic alkylation reaction of isobutane is carried out in a continuous apparatus using acidic ionic liquid as catalyst, wherein the cation is provided by $Et_3NHCl$ and the anion is provided by $AlCl_3$. The ionic liquid is added at an amount of 30kg. The reaction pressure is 0.5MPa and the reaction temperature is 30°C. The reaction feed is a mixture of isobutane, 2-butene, iso-butene and 1-butene with a molar ratio between alkane and alkene of 10:1. The feed flowrate is of 12kg/h, and the alkylate is produced at a rate of 2.4kg/h.
The ionic liquid is separated from the reaction system continuously at a rate of 3kg/h and supplied into a mixer together with 2-chloro-2-methyl propane at a rate of 6g/h, and is reinjected back to the reaction system continuously after mixed. The flow diagram of the reaction may refer to Fig.2.

Collecting the alkylation product and analysing the composition thereof, and the results are shown in table 12. As can be seen, when the feed processed by per kilogram of the ionic liquid catalyst is up to 1000 kg, the catalyst is still not deactivated and the selectivity of the targeted product C8 fractions keeps unchanged.

Table 12 Continuous evaluation on catalytic alkylation by the ionic liquid with 2-chloro-2-methyl propane supplied thereinto semi-continuously

| Processed feed, kg/kg ionic liquid | Butene conversion, % | Selectivity of C8 fractions, wt% | TMP/DMH |
| --- | --- | --- | --- |
| 200 | 100 | 81 | 12 |
| 400 | 100 | 82 | 12 |
| 600 | 100 | 81 | 12 |
| 800 | 100 | 81 | 12 |
| 1000 | 100 | 81 | 12 |

Example 8:

Catalytic alkylation reaction of isobutane is carried out in a continuous apparatus using acidic ionic liquid as catalyst, wherein the cation is provided by $Et_3NHCl$ and the anion is provided by $AlCl_3$ and CuCl. The ionic liquid is added at an amount of 200g. The reaction pressure is 0.5MPa and the reaction temperature is 25°C. The reaction feed is a mixture of isobutane and 2-butene with a molar ratio between alkane and alkene of 20:1. The feed flowrate is of 500g/h, and the alkylate is produced at a rate of 50g/h.

The ionic liquid is separated from the reaction system continuously at a rate of 10g/h and supplied into a mixer together with hydrogen chloride at a rate of0.03g/h, and and is reinjected back to the reaction system continuously after mixed. The flow diagram of the reaction may refer to Fig.2.

Collecting the alkylation product and analysing the composition thereof, and the results are shown in table 13.

As can be seen, when the feed processed by per gram of the ionic liquid catalyst is up to 1000 g, the catalyst is still not deactivated and the selectivity of the targeted product C8 fractions keeps unchanged.

Table 13 Continuous evaluation on catalytic alkylation by the ionic liquid with hydrogen chloride supplied thereinto semi-continuously

| Processed feed, kg/kg ionic liquid | Butene conversion, % | Selectivity of C8 fractions, wt% | TMP/DMH |
| --- | --- | --- | --- |
| 200 | 100 | 85 | 14 |
| 400 | 100 | 86 | 14 |
| 600 | 100 | 86 | 15 |
| 800 | 100 | 86 | 15 |
| 1000 | 100 | 86 | 15 |

Comparative Example 2:

Catalytic alkylation reaction of isobutane is carried out in a continuous apparatus using acidic ionic liquid as catalyst, wherein the cation is provided by $Et_3NHCl$ and the anion is provided by $AlCl_3$ and CuCl. The ionic liquid is added at an amount of 200g. The reaction pressure is 0.5MPa and the reaction temperature is 25°C. The reaction feed is a mixture of isobutane and 2-butene with a molar ratio between alkane and alkene of 20:1. The feed flowrate is of 500g/h, and the alkylate is produced at a rate of 50g/h.

The ionic liquid is separated from the reaction system continuously at a rate of 10kg/h and supplied into a mixer together with hydrogen chloride at a rate of 0.002g/h, and is reinjected back to the reaction system continuously after mixed.

Collecting the alkylation product and analysing the composition thereof, and the results are shown in table 14. When the feed processed by per gram of the ionic liquid catalyst is up to 120 g, the catalyst is deactivated. Compared with Example 8, the lifetime of the ionic liquid is not prolonged effectively.

Table 14 Continuous evaluation on catalytic alkylation by the ionic liquid with hydrogen chloride supplied thereinto semi-continuously

| Processed feed, kg/kg ionic liquid | Butane conversion, % | Selectivity of C8 fractions, wt% | TMP/DMH |
|---|---|---|---|
| 20 | 100 | 85 | 14 |
| 40 | 100 | 86 | 15 |
| 60 | 100 | 86 | 15 |
| 80 | 100 | 86 | 15 |
| 100 | 47 | 75 | 12 |
| 120 | 0 | - | - |

Finally, it should be noted that all the above examples is intend to describe the technical solutions of the present invention only and should not be considered as limitations of the present invention. All the modification or equivalents of the technical solutions of the present invention disclosed herein by those skilled in the art should fall into the scope of the present invention claimed in appended claims.

**Claims**

1. A process for regenerating and maintaining the activity of an ionic liquid catalyst, which is used to catalyze the alkylation reaction for producing alkylate, said process is **characterized by** supplying hydrogen halide or halogenated hydrocarbon to the acidic ionic liquid catalyst or the reaction feed during the alkylation reaction.

2. The process according to claim 1, wherein the hydrogen halide is supplied at an amount of 0.01-1wt% of the produced alkylate; and the halogenated hydrocarbon is supplied at an amount of 0.01-1wt% of the produced alkylate calculated as the mass of the halogen contained therein.

3. The process according to claim 1, wherein the acidic ionic liquid has a cation derived from hydrohalide of alkyl amine, hydrohalide of imidazole or hydrohalide of pyridine and an anion derived from one or more metallic compounds.

4. The process according to claim 3, wherein the acidic ionic liquid has anion derived from two or more metallic compounds, of which at least one metallic compound is aluminum chloride or aluminum bromide and the other metallic compounds are halide, sulphate or nitrate of copper, iron, zinc, nickel, titanium or silver.

5. The process according to claim 1, wherein the hydrogen halide is hydrogen chloride or hydrogen bromide, and the halogenated hydrocarbon is chlorohydrocarbon or bromohydrocarbon composing at least 4 carbons.

6. The process according to claim 5, wherein the halogenated hydrocarbon is halogenated alkane having 4-8 carbons, and in the structure of the halogenated hydrocarbon the carbon having halogen atom attached thereto is secondary or tertiary carbon.

7. A process for producing alkylate by alkylation reaction using isobutene and C4 alkene as reaction feed and acidic ionic liquid as catalyst, said process is **characterized by** supplying hydrogen halide or halogenated hydrocarbon to the acidic ionic liquid catalyst or the reaction feed during the alkylation reaction to regenerate the acidic ionic liquid catalyst.

8. The process according to claim 7, wherein the hydrogen halide is supplied at an amount of 0.01-1wt% of the produced alkylate; and the halogenated hydrocarbon is supplied at an amount of 0.01-1wt% of the produced alkylate calculated as the mass of the halogen contained therein.

9. The process according to claim 7, wherein the acidic ionic liquid has a cation derived from hydrohalide of alkyl amine, hydrohalide of imidazole or hydrohalide of pyridine and an anion derived from one or more metallic compounds.

**10.** The process according to claim 9, wherein the acidic ionic liquid has anion derived from two or more metallic compounds, of which at least one metallic compound is aluminum chloride or aluminum bromide and the other metallic compounds are halide, sulphate or nitrate of copper, iron, zinc, nickel, titanium or silver.

**11.** The process according to claim 7, wherein the hydrogen halide is hydrogen chloride or hydrogen bromide, and the halogenated hydrocarbon is chlorohydrocarbon or bromohydrocarbon comprising at least 4 carbons.

**12.** The process according to claim 11, wherein the halogenated hydrocarbon is halogenated alkane having 4-8 carbons, and in the structure of the halogenated hydrocarbon the carbon having halogen atom attached thereto is secondary or tertiary carbon.

**13.** The process according to claim 7, wherein C4 alkene comprises 1-butene, 2-butene, iso-butene or mixture thereof.

**14.** The process according to claim 7 or 8, wherein supplying hydrogen halide or halogenated hydrocarbon to the acidic ionic liquid catalyst or the reaction feed comprises the following steps:

(i) Stopping feeding after the catalytic alkylation reaction using the acidic ionic liquid is proceeding for a period;
(ii) Supplying hydrogen halide or halogenated hydrocarbon to the acidic ionic liquid catalyst and intensively mixing the same;
(iii) Continuing the catalytic alkylation reaction using the acidic ionic liquid with hydrogen halide or halogenated hydrocarbon supplied thereinto and repeating steps (i) and (ii).

**15.** The process according to claim 7 or 8, wherein supplying hydrogen halide or halogenated hydrocarbon to the acidic ionic liquid catalyst or the reaction feed comprises the following step:

Continuously supplying hydrogen halide or halogenated hydrocarbon to the acidic ionic liquid catalyst or the reaction feed while the alkylation reaction is proceeding.

**16.** The process according to claim 7 or 8, wherein supplying hydrogen halide or halogenated hydrocarbon to the acidic ionic liquid catalyst or the reaction feed comprises the following steps:

(i) Separating a part of the acidic ionic liquid catalyst while the catalytic alkylation reaction using the acidic ionic liquid catalyst is proceeding;
(ii) Supplying hydrogen halide or halogenated hydrocarbon to the separated acidic ionic liquid catalyst and intensively mixing the same;
(iii) Reinjecting the acidic ionic liquid with hydrogen halide or halogenated hydrocarbon supplied thereinto back to the reaction system and repeating steps (i) and (ii).

hydrogen
halide or          reaction
halogenated        feed          ┌──────────┐
hydrocarbon                      │          │         alkylation
                                 │ reactor  │───────→ product
                                 │          │
                                 └──────────┘
                                      ↑
                                    ionic
                                    liguid

Fig. 1

                   ┌──────────┐
reaction           │          │          alkylation
feed               │ reactor  │────────→  product
                   │          │
                   └──────────┘           hydrogen halide
                        ↑                 or halogenated
                      ionic               hydrocarbon
                      liguid    ┌───────┐
                                │ mixer │←─────────
                      regenerated └───────┘
                      ionic liguid

Fig. 2

| | | INTERNATIONAL SEARCH REPORT | International application No. |
|---|---|---|---|

<table>
<tr><td colspan="4"><b>INTERNATIONAL SEARCH REPORT</b></td><td>International application No.<br>PCT/CN2010/001066</td></tr>
</table>

# INTERNATIONAL SEARCH REPORT

International application No.

PCT/CN2010/001066

**A.   CLASSIFICATION OF SUBJECT MATTER**

See extra sheet

According to International Patent Classification (IPC) or to both national classification and IPC

**B.   FIELDS SEARCHED**

Minimum documentation searched (classification system followed by classification symbols)

IPC：C07C 2/-; C10G 50/-; B01J 38/-; B01J 31/-

Documentation searched other than minimum documentation to the extent that such documents are included in the fields searched

Electronic data base consulted during the international search (name of data base and, where practicable, search terms used)

CPRS; CNKI; WPI; EPODOC; CA; STN

ionic liquid, alkylat+, hydrogen halide, hydrogen chloride, hydrogen bromide, halogenated hydrocarbon, alkane

**C.   DOCUMENTS CONSIDERED TO BE RELEVANT**

| Category* | Citation of document, with indication, where appropriate, of the relevant passages | Relevant to claim No. |
|---|---|---|
| PX | CN101619010A (UNIV CHINA PETROLEUM (BEIJING)), 06 Jan. 2010(06.01.2010), claims 1-16 | 1-16 |
| X | WO2008/076722A1 (CHEVRON U.S.A. INC.), 26 Jun. 2008 (26.06.2008), page 5, lines 3-7; page 6, line 26, lines 22-24; page 8, lines 6-7, lines 13-19, lines 29-32; page 9, lines 4-5; page 14, lines 7-10 and table 1 | 1-16 |
| A | CN1500764A (UNIV PETROLEUM (BEIJING)), 02 Jun. 2004(02.06.2004), the whole document | 1-16 |

☐ Further documents are listed in the continuation of Box C.          ☒ See patent family annex.

| *       Special categories of cited documents: | "T"   later document published after the international filing date or priority date and not in conflict with the application but cited to understand the principle or theory underlying the invention |
|---|---|
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim (S) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"document member of the same patent family |

| Date of the actual completion of the international search | Date of mailing of the international search report |
|---|---|
| 21 Sep.2010(21.09.2010) | **28 Oct. 2010 (28.10.2010)** |
| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br>LI, Yong<br>Telephone No. (86-10)62084574 |

Form PCT/ISA /210 (second sheet) (July 2009)

<div align="center">

**INTERNATIONAL SEARCH REPORT**
Information on patent family members

</div>

| | | International application No. |
|---|---|---|
| | | PCT/CN2010/001066 |

| Patent Documents referred in the Report | Publication Date | Patent Family | Publication Date |
|---|---|---|---|
| CN101619010A | 06.01.2010 | none | |
| WO2008/076722A1 | 26.06.2008 | US2008142413A | 19.06.2008 |
| | | US7531707B | 12.05.2009 |
| | | AU2007334057A | 26.06.2008 |
| | | KR20090087489A | 17.08.2009 |
| | | DE112007003012T | 05.11.2009 |
| | | CN101622214A | 06.01.2010 |
| CN1500764A | 02.06.2004 | CN1203032C | 25.05.2005 |
| | | JP2004161763A | 10.06.2004 |
| | | JP4153864B2B | 24.09.2008 |
| | | US2004133056A | 08.07.2004 |
| | | US7285698B | 23.10.2007 |

Form PCT/ISA /210 (patent family annex) (July 2009)

## **INTERNATIONAL SEARCH REPORT**

International application No.

*PCT/CN2010/001066*

Continuation of:

(1) CLASSIFICATION OF SUBJECT MATTER

C07C 2/60 (2006.01) i

C10G 50/00 (2006.01) i

B01J 38/54 (2006.01) i

B01J 38/48 (2006.01) i

B01J 31/02 (2006.01) n

Form PCT/ISA /210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7285698 B
- US 20040133056 A1
- CN 02149296
- CN 200710063459
- CN 200680051282
- CN 200680052353X
- US 20070142211 A
- US 20070142214 A